# EUROPEAN PATENT SPECIFICATION

(11) **EP 2 927 216 B1**
(45) Date of publication and mention of the grant of the patent: **24.10.2018**
(21) Application number: 13859004.7
(22) Date of filing: 27.11.2013
(51) Int. Cl.: C07D 221/14, C09K 3/00, G03F 7/004

(54) **NOVEL SULFONIC ACID DERIVATIVE COMPOUND, PHOTOACID GENERATOR, CATIONIC POLYMERIZATION INITIATOR, RESIST COMPOSITION, AND CATIONICALLY POLYMERIZABLE COMPOSITION**
NEUARTIGE SULFONSÄUREDERIVATVERBINDUNG, FOTOSÄUREGENERATOR, KATIONISCHER POLYMERISATIONSINITIATOR, LACKZUSAMMENSETZUNG UND KATIONISCH POLYMERISIERBARE ZUSAMMENSETZUNG
COMPOSÉ DE TYPE DÉRIVÉ D'ACIDE SULFONIQUE INÉDIT, GÉNÉRATEUR PHOTOACIDE, INITIATEUR DE POLYMÉRISATION CATIONIQUE, COMPOSITION DE RÉSINE PHOTOSENSIBLE ET COMPOSITION CATIONIQUEMENT POLYMÉRISABLE

(30) Priority: 28.11.2012 JP 2012259874
(43) Date of publication of application: 07.10.2015
(73) Proprietor: Adeka Corporation, Tokyo 116-0012 (JP)
(72) Inventor: YANAGISAWA, Satoshi, Tokyo 116-0012 (JP); FUJITA, Shohei, Tokyo 116-0012 (JP); MINAMISHIMA, Takuya, Tokyo 116-0012 (JP); SHIGENO, Koichi, Tokyo 116-0012 (JP)
(74) Representative: Gill Jennings & Every LLP
(86) International application number: PCT/JP2013/081939
(87) International publication number: WO 2014/084269

(56) References cited:
- WO-A1-2011/087011
- JP-A- H0 519 505
- JP-A- H0 525 136
- JP-A- H0 867 827
- JP-A- H08 259 542
- JP-A- 2004 217 748
- JP-A- 2004 217 748
- JP-A- 2010 053 121
- US-A1- 2002 198 385
- US-A1- 2010 028 807
- US-A1- 2011 229 821
- US-A1- 2012 289 697
- US-A1- 2013 134 426

## Description

### TECHNICAL FIELD

The present invention relates to a novel sulfonic acid derivative compound. More particularly, the present invention relates to a sulfonic acid derivative compound that is useful as a photoacid generator and as a cationic polymerization initiator.

### BACKGROUND ART

Sulfonyloxyimides having a naphthalimino group which is a radioactive functional group are substances that generate an acid when irradiated with an energy beam such as light, and they are used, for example, as a photoacid generator in photolithography resist compositions used for formation of an electronic circuit such as a semiconductor, and as a cationic polymerization initiator in photo-polymerizable compositions such as resin compositions for stereolithography, paints, coatings, adhesives and inks.

Patent Document 1 discloses a curable composition comprising an acid-curable resin and a latent curing catalyst represented by the Formula (II). It is disclosed that, in the Formula (II), R¹ to R⁴ which are substituents of the naphthalene skeleton are each a hydrogen atom, an alkyl group having 1 to 8 carbon atoms, an alkoxy group having 1 to 4 carbon atoms, an alkylthio group having 1 to 12 carbon atoms, a nitro group or a halogen atom. However, Patent Document 1 discloses only the case where R¹ to R⁴ are hydrogen atoms, and it offers neither disclosure nor suggestion with regard to the differences in properties and performance that are caused by differences in the types of these substituents, the number of substitutions, the positions of substitutions and the like.

Patent Document 2 discloses a photoresist comprising a sulfonyloxyimide represented by the Formula (I) as a sulfonic acid precursor, which photoresist is used in an ultraviolet radiation, electron beam or X-ray exposure apparatus. In Patent Document 2, as naphthalimides, naphthalimide, 3-nitronaphthalimide, 4-nitronaphthalimide, 4-chloronaphthalimide and 4-bromonaphthalimide are disclosed.

Patent Document 3 discloses an active ray-curable ink composition comprising a sulfonic acid generator represented by the Formula (A-1). As R₁ and R₂ which are substituents of the naphthalene skeleton in the Formula (A-1), alkyl groups, alkoxy groups, a carbonyl group, a phenylthio group, halogen atoms, a cyano group, a nitro group and a hydroxy group are disclosed.

Patent Document 4 discloses an undercoat composition for a photoresist. A fluorinated sulfonyloxyimide having a naphthalimino group is disclosed as a photoactive compound, and (C₁-C₈) alkyl and (C₁-C₈) alkoxy are disclosed as substituents of a naphthalene skeleton. However, Patent Document offers neither disclosure nor suggestion with regard to the differences in properties and performance that are caused by differences in the types of substitutions, the positions of substitutions and the like.

US2012/289697 and US2010/028807 also disclose sulfonic acid derivatives which can be used in a resist composition. As a light source for a photoacid generator used in photoresists or a cationic polymerization initiator used in compositions for stereolithography, adhesives, inks and the like, a far-ultraviolet ray such as EUV (Extreme Ultra-Violet), X-ray, F₂, ArF, KrF, I-line, H-line or G-line, an electron beam or a radioactive ray is often used. The use of these light sources is advantageous for those materials that show large absorption at a wavelength of 365 nm. Further, from the standpoint of coping with high-precision patterning as well as shortening of the process, it is desired that, in a photoresist or cationic polymerization system, an adequate amount of an acid generator be incorporated or an acid generator having favorable acid generation rate be used. Accordingly, there is a demand for an acid generator which shows high solubility to an organic solvent and/or has sufficient acid generation rate.

### RELATED ART DOCUMENTS

### PATENT DOCUMENTS

Patent Document 1: Japanese Unexamined Patent Application Publication No. S57-151651 (Claim 1)
Patent Document 2: Japanese Unexamined Patent Application Publication (Translation of PCT Application) No. H8-501890 (Claim 2)
Patent Document 3: Japanese Unexamined Patent Application Publication No. 2004-217748 (Claim 1)
Patent Document 4: Japanese Unexamined Patent Application Publication (Translation of PCT Application) No. 2009-516207 (paragraphs [0029] and [0034])

### SUMMARY OF THE INVENTION

### PROBLEMS TO BE SOLVED BY THE INVENTION

In view of the above, an object of the present invention is to provide a compound which shows large absorption for light having a wavelength of 365 nm and has good acid generation rate; and a photoacid generator, cationic polymerization initiator, resist composition and cationically polymerizable composition that use the compound.

### MEANS FOR SOLVING THE PROBLEMS

In order to solve the above-described problems, the present inventors intensively studied and discovered that the problems can be solved by a sulfonic acid derivative compound having a specific structure, thereby completing the present invention.

That is, the sulfonic acid derivative compound of the present invention is characterized in that it is represented by the following Formula (I): 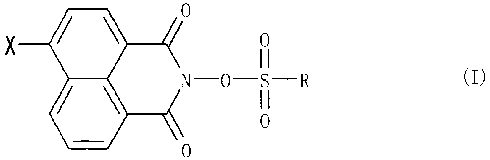 (wherein, X represents a linear or branched alkyl group having 3 to 8 carbon atoms; and R represents an aliphatic hydrocarbon group having 1 to 18 carbon atoms, an aryl group having 6 to 20 carbon atoms, an arylalkyl group having 7 to 20 carbon atoms, an aryl group having 7 to 20 carbon atoms which is substituted with an acyl group, an alicyclic hydrocarbon group having 3 to 12 carbon atoms, 10-camphoryl group or a group represented by the following Formula (II), which aliphatic hydrocarbon group, aryl group, arylalkyl group or alicyclic hydrocarbon group is unsubstituted or substituted with a group selected from a halogen atom, a halogenated alkyl group having 1 to 4 carbon atoms, an alkoxy group having 1 to 18 carbon atoms and an alkylthio group having 1 to 18 carbon atoms) 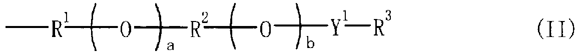 (wherein, Y¹ represents a single bond or an alkanediyl group having 1 to 4 carbon atoms; R¹ and R² each independently represent an alkanediyl group having 2 to 6 carbon atoms, a halogenated alkanediyl group having 2 to 6 carbon atoms, an arylene group having 6 to 20 carbon atoms or a halogenated arylene group having 6 to 20 carbon atoms; R³ represents a linear or branched alkyl group having 1 to 18 carbon atoms, a halogenated linear or branched alkyl group having 1 to 18 carbon atoms, an alicyclic hydrocarbon group having 3 to 12 carbon atoms, an aryl group having 6 to 20 carbon atoms, a halogenated aryl group having 6 to 20 carbon atoms, an arylalkyl group having 7 to 20 carbon atoms or a halogenated arylalkyl group having 7 to 20 carbon atoms; a and b each represent 0 or 1; and one of a and b is 1).

In the sulfonic acid derivative compound of the present invention, it is preferred that the X in the Formula (I) be an alkyl group having 4 carbon atoms.

In the sulfonic acid derivative compound of the present invention, it is preferred that the R in the Formula (I) be a perfluoroalkyl group having 1 to 8 carbon atoms.

The photoacid generator of the present invention is characterized by being composed of any one of the above-described sulfonic acid derivative compounds.

The cationic polymerization initiator of the present invention is characterized by being composed of any one of the above-described sulfonic acid derivative compounds.

The resist composition of the present invention is characterized by comprising the photoacid generator.

The cationically polymerizable composition of the present invention is characterized by comprising the cationic polymerization initiator.

### EFFECTS OF THE INVENTION

According to the present invention, a compound which shows large absorption for light having a wavelength of 365 nm and has good acid generation rate, as well as a photoacid generator and a cationic polymerization initiator that use the compound, can be provided.

### MODE FOR CARRYING OUT THE INVENTION

The present invention will now be described in detail based on embodiments thereof.

First, the sulfonic acid derivative compound of the present invention, which is represented by the Formula (I), will be described.

The compound of the present invention is structurally characterized by having a linear or branched alkyl group having 3 to 8 carbon atoms at a specific position of the naphthalimide skeleton of photosensitive group (at 4-position of the naphthalene structure). This structure increases the absorption (molar extinction coefficient, ε) at a wavelength of 365 nm, imparts the compound with good acid generation rate and improves the solubility to organic solvents. When the X is a hydrogen atom, such effects cannot be obtained. For example, when the X has more than 14 carbon atoms, although the solubility is increased, the molecular weight of the compound is increased and an adequate acid generation rate cannot be maintained for the amount of use. In addition, it is known that the compound does not show sufficient absorption for light having a wavelength of 365 nm when an alkyl group exists at the 3-position of the naphthalene structure.

Examples of the X include propyl, isopropyl, 1-butyl, 2-butyl, isobutyl, tert-butyl, 1-pentyl, isopentyl, tert-pentyl, neopentyl, 1-hexyl, 2-hexyl, 3-hexyl, heptyl, 2-heptyl, 3-heptyl, isoheptyl, tert-heptyl, 1-octyl, isooctyl, tert-octyl and 2-ethylhexyl. X is an alkyl group having 3 to 8 carbon atoms, preferably an alkyl group having 4 carbon atoms because these alkyl groups have both good solubility and good acid generation rate. A 1-butyl group is still more preferred because the material is inexpensive and has good yield and low production cost. It is yet still more preferred that the X be an unsubstituted alkyl group.

In the Formula (I), the R represents an aliphatic hydrocarbon group having 1 to 18 carbon atoms, an aryl group having 6 to 20 carbon atoms, an arylalkyl group having 7 to 20 carbon atoms, an aryl group having 7 to 20 carbon atoms which is substituted with an acyl group, an alicyclic hydrocarbon group having 3 to 12 carbon atoms, 10-camphoryl group, or a group represented by the Formula (II). Among these groups, the aliphatic hydrocarbon group, the aryl group and the arylalkyl group are not required to have a substituent but are optionally substituted with a halogen atom or a group selected from a halogenated alkyl group having 1 to 4 carbon atoms, an alkoxy group having 1 to 18 carbon atoms and an alkylthio group having 1 to 18 carbon atoms. Examples of the halogen atom, which is the substituent, include chlorine, bromine, iodine and fluorine. Examples of the halogenated alkyl group include trifluoromethyl group.

Examples of the alkoxy group having 1 to 18 carbon atoms include methoxy, ethoxy, propoxyl, butoxy, tert-butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy and octadecyloxy.

Examples of the alkylthio group having 1 to 18 carbon atoms include methylthio, ethylthio, propylthio, isopropylthio, butylthio, sec-butylthio, tert-butylthio, isobutylthio, amylthio, isoamylthio, tert-amylthio, hexylthio, heptylthio, isoheptylthio, tert-heptylthio, octylthio, isooctylthio, tert-octylthio, 2-ethylhexylthio, nonylthio, decylthio, undecylthio, dodecylthio, tridecylthio, tetradecylthio, pentadecylthio, hexadecylthio, heptadecylthio and octadecylthio.

Examples of the aliphatic hydrocarbon group having 1 to 18 carbon atoms which may be represented by the R include an alkenyl group, an alkyl group, a group in which a methylene group in an alkyl group is substituted with an alicyclic hydrocarbon group, a group in which a proton of a methylene group in an alkyl group is substituted with an alicyclic hydrocarbon group, and a group in which an alicyclic hydrocarbon exists at a terminal of an alkyl group. Examples of the alkenyl group include allyl and 2-methyl-2-propenyl, and examples of the alkyl group include methyl, ethyl, propyl, isopropyl, butyl, sec-butyl, tert-butyl, isobutyl, amyl, isoamyl, tert-amyl, hexyl, 2-hexyl, 3-hexyl, heptyl, 2-heptyl, 3-heptyl, isoheptyl, tert-heptyl, octyl, isooctyl, tert-octyl, 2-ethylhexyl, nonyl, isononyl, decyl, dodecyl, tridecyl, tetradecyl, pentadecyl, hexadecyl, heptadecyl and octadecyl. Examples of the alicyclic hydrocarbon group include the same ones as described below.

Examples of the aliphatic hydrocarbon group having 1 to 18 carbon atoms which is substituted with a halogen atom include halogenated alkyl groups such as trifluoromethyl, pentafluoroethyl, 2-chloroethyl, 2-bromoethyl, heptafluoropropyl, 3-bromopropyl, nonafluorobutyl, tridecafluorohexyl, heptadecafluorooctyl, 2,2,2-trifluoroethyl, 1,1-difluoroethyl, 1,1-difluoropropyl, 1,1,2,2-tetrafluoropropyl, 3,3,3-trifluoropropyl, 2,2,3,3,3-pentafluoropropyl, norbornyl-1,1-difluoroethyl, norbornyltetrafluoroethyl, adamantane-1,1,2,2-tetrafluoropropyl and bicyclo [2.2.1]heptane-tetrafluoromethyl.

Examples of the aliphatic hydrocarbon having 1 to 18 carbon atoms which is substituted with an alkylthio group include 2-methylthioethyl, 4-methylthiobutyl and 4-butylthioethyl, and examples of the aliphatic hydrocarbon having 1 to 18 carbon atoms which is substituted with a halogen atom and an alkylthio group having 1 to 18 carbon atoms include 1,1,2,2-tetrafluoro-3-methylthiopropyl.

Examples of the aryl group having 6 to 20 carbon atoms include phenyl, naphthyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-tert-butylphenyl, 4-hexylphenyl, 4-cyclohexylphenyl, 4-octylphenyl, 4-(2-ethylhexyl)phenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4-di-tert-butylphenyl, 2,5-di-tert-butylphenyl, 2,6-di-tert-butylphenyl, 2,4-di-tert-pentylphenyl, 2,5-di-tert-amylphenyl, 2,5-di-tert-octylphenyl, cyclohexylphenyl, biphenyl, 2,4,5-trimethylphenyl, 2,4,6-trimethylphenyl and 2,4,6-triisopropylphenyl.

Examples of the aryl group having 6 to 20 carbon atoms which is substituted with a halogen atom include pentafluorophenyl, chlorophenyl, dichlorophenyl, trichlorophenyl, 2,4-bis(trifluoromethyl)phenyl and bromoethylphenyl.

Examples of the aryl group having 6 to 20 carbon atoms which is substituted with an alkylthio group having 1 to 18 carbon atoms include 4-methylthiophenyl, 4-butylthiophenyl, 4-octylthiophenyl and 4-dodecylthiophenyl. Examples of the aryl group having 6 to 20 carbon atoms which is substituted with a halogen atom and an alkylthio group having 1 to 18 carbon atoms include 1,2,5,6-tetrafluoro-4-methylthiophenyl, 1,2,5,6-tetrafluoro-4-butylthiophenyl and 1,2,5,6-tetrafluoro-4-dodecylthiophenyl.

Examples of the arylalkyl group having 7 to 20 carbon atoms include benzyl, phenethyl, 2-phenylpropane-2-yl, diphenylmethyl, triphenylmethyl, styryl and cinnamyl.

Examples of the arylalkyl group substituted with a halogen atom include pentafluorophenylmethyl, phenyldifluoromethyl, 2-phenyl-tetrafluoroethyl and 2-(pentafluorophenyl)ethyl. Examples of the arylalkyl group having 7 to 20 carbon atoms which is substituted with an alkylthio group having 1 to 18 carbon atoms include p-methylthiobenzyl. Examples of the arylalkyl group having 7 to 20 carbon atoms which is substituted with a halogen atom and an alkylthio group having 1 to 18 carbon atoms include 2,3,5,6-tetrafluoro-4-methylthiophenylethyl.

The carbon number of the aryl group having 7 to 20 carbon atoms which is substituted with an acyl group include the carbon atoms of the acyl group. Examples of such aryl group include acetylphenyl, acetylnaphthyl, benzoylphenyl, 1-anthraquinolyl and 2-anthraquinolyl.

Examples of the alicyclic hydrocarbon group include, mentioning them in terms of the name of the cycloalkane constituting the respective alicyclic hydrocarbon groups, cyclopropane, cyclobutane, cyclopentane, cyclohexane, cycloheptane, cyclooctane, cyclodecane, bicyclo[2.1.1]hexane, bicyclo[2.2.1]heptane, bicyclo[3.2.1]octane, bicyclo[2.2.2]octane and adamantane.

The Formula (II) represents an ether group. In the Formula (II), examples of the alkanediyl group having 1 to 4 carbon atoms which is represented by Y¹ include methylene, ethylene, propane-1,3-diyl, propane-1,2-diyl, butylene, butane-1,3-diyl, butane-2,3-diyl and butane-1,2-diyl. Examples of the alkanediyl group having 2 to 6 carbon atoms which is represented by the R¹ and R² include ethylene, propane-1,3-diyl, propane-1,2-diyl, butylene, butane-1,3-diyl, butane-2,3-diyl, butane-1,2-diyl, pentane-1,5-diyl, pentane-1,3-diyl, pentane-1,4-diyl, pentane-2,3-diyl, hexane-1,6-diyl, hexane-1,2-diyl, hexane-1,3-diyl, hexane-1,4-diyl, hexane-2,5-diyl, hexane-2,4-diyl and hexane-3,4-diyl. The halogenated alkanediyl group having 2 to 6 carbon atoms is one in which at least one proton of any one of the above-described alkanediyl groups having 1 to 6 carbon atoms is substituted with a halogen atom. Examples of the halogen atom include chlorine, bromine, iodine and fluorine. Examples of the halogenated alkanediyl group having 2 to 6 carbon atoms include tetrafluoroethylene, 1,1-difluoroethylene, 1-fluoroethylene, 1,2-difluoroethylene, hexafluoropropane-1,3-diyl, 1,1,2,2-tetrafluoropropane-1,3-diyl and 1,1,2,2-tetrafluoropentane-1,5-diyl.

In the Formula (II), examples of the arylene group having 6 to 20 carbon atoms which is represented by the R¹ and R² include 1,2-phenylene, 1,3-phenylene, 1,4-phenylene, 2,5-dimethyl-1,4-phenylene, 4,4'-biphenylene, diphenylmethane-4,4'-diyl, 2,2-diphenylpropane-4,4'-diyl, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl and naphthalene-2,7-diyl, and the halogenated arylene group having 6 to 20 carbon atoms is one in which at least one proton of any one of the above-described arylene groups having 6 to 20 carbon atoms is substituted with a halogen atom. Examples of the halogen atom include chlorine, bromine, iodine and fluorine. Examples of the halogenated arylene group having 6 to 20 carbon atoms include tetrafluorophenylene.

In the Formula (II), examples of the alkyl group having 1 to 18 carbon atoms which is represented by the R³ include methyl, ethyl, propyl, isopropyl, butyl, sec-butyl, tert-butyl, isobutyl, amyl, isoamyl, tert-amyl, hexyl, 2-hexyl, 3-hexyl, heptyl, 2-heptyl, 3-heptyl, isoheptyl, tert-heptyl, octyl, isooctyl, tert-octyl, 2-ethylhexyl, nonyl, isononyl, decyl, dodecyl, tridecyl, tetradecyl, pentadecyl, hexadecyl, heptadecyl and octadecyl, and the halogenated alkyl group having 1 to 18 carbon atoms is one in which at least one proton of any one of the above-described alkyl groups having 1 to 18 carbon atoms is substituted with a halogen atom. Examples of the halogen atom include chlorine, bromine, iodine and fluorine. Examples of the halogenated alkyl group having 1 to 18 carbon atoms include halogenated alkyl groups such as trifluoromethyl, pentafluoroethyl, heptafluoropropyl, nonafluorobutyl, tridecafluorohexyl, heptadecafluorooctyl, 2,2,2-trifluoroethyl, 1,1-difluoroethyl, 1,1-difluoropropyl, 1,1,2,2-tetrafluoropropyl, 3,3,3-trifluoropropyl, 2,2,3,3,3-pentafluoropropyl and 1,1,2,2-tetrafluorotetradecyl.

In the Formula (II), examples of the alicyclic hydrocarbon group having 3 to 12 carbon atoms which is represented by the R³ include, mentioning them in terms of the name of the cycloalkane constituting the respective alicyclic hydrocarbon groups, cyclopropane, cyclobutane, cyclopentane, cyclohexane, cycloheptane, cyclooctane, cyclodecane, bicyclo[2.1.1]hexane, bicyclo[2.2.1]heptane, bicyclo[3.2.1]octane, bicyclo[2.2.2]octane and adamantane.

In the Formula (II), examples of the aryl group having 6 to 20 carbon atoms, halogenated aryl group having 6 to 20 carbon atoms, arylalkyl group having 7 to 20 carbon atoms or halogenated arylalkyl group having 7 to 20 carbon atoms which is represented by the R³ include the same groups as those exemplified above for the R.

A group preferred as the Formula (II) is a group having a total of 2 to 18 carbon atoms in which fluorine is bound to the carbon atom adjacent to a sulfur atom of a group represented by the R¹, because such a group has good acid generation capacity, cationic polymerizability and the like.

Specific examples of the compound of the present invention include the following Compound Nos. 1 to 43. Compounds Nos. 20, 23, 24, 28, 40, 41 and 42 are comparative compounds.

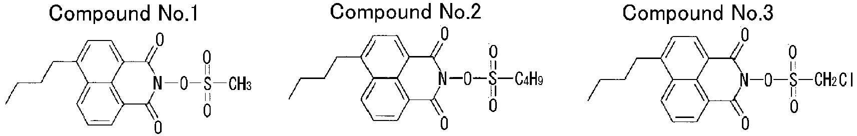

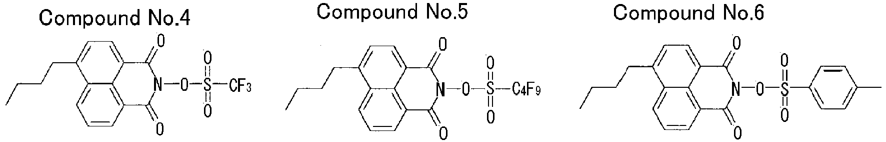

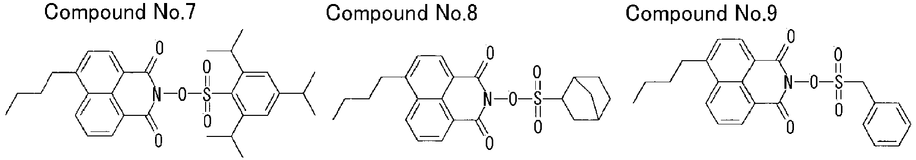

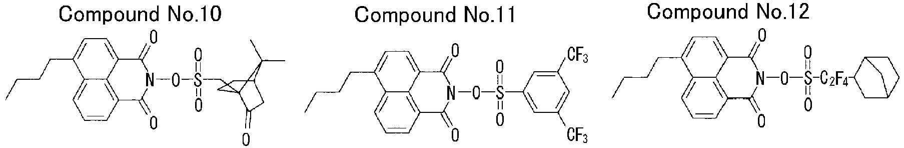

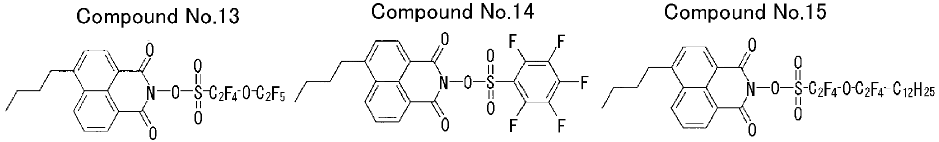

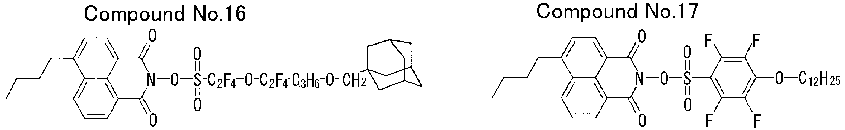

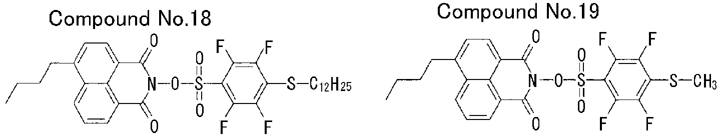

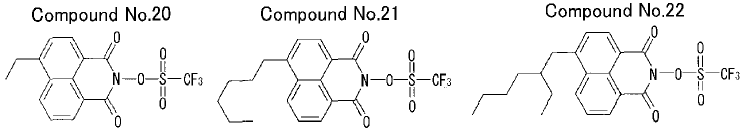

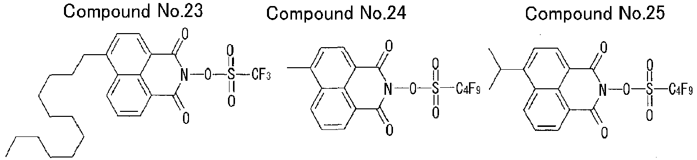

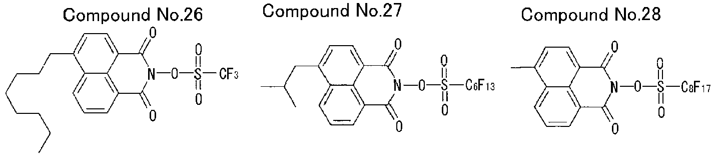

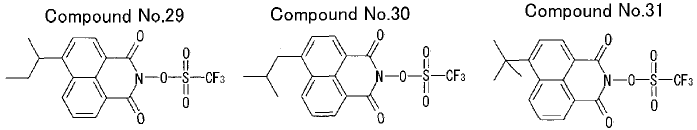

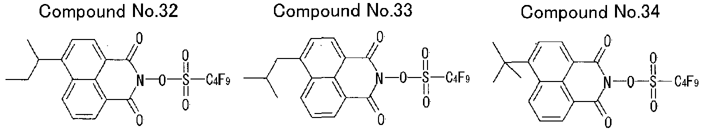

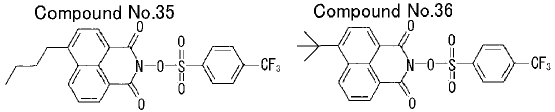

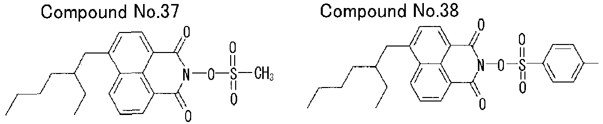

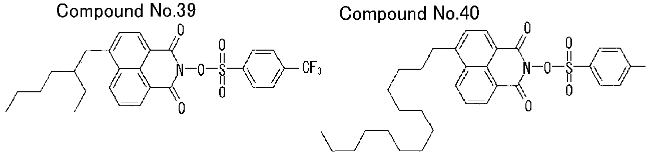

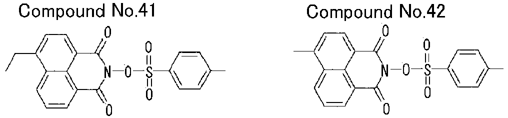

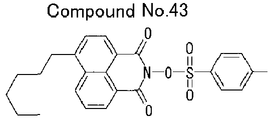

The R in the Formula (I) may be selected such that it releases an appropriate organic sulfonic acid according to the intended use. For high-sensitive and high-precision patterning, perfluoroalkanesulfonic acid which has high acid strength and provides high sensitivity is most useful. Accordingly, in the compound of the present invention, the R is preferably a perfluoroalkyl group having 1 to 8 carbon atoms.

A method of producing the sulfonic acid derivative compound of the present invention is not particularly restricted, and a well-known chemical reaction can be applied to synthesize the sulfonic acid derivative compound of the present invention. For example, a method of synthesizing a sulfonic acid derivative compound using a bromide as a starting substance can be employed. 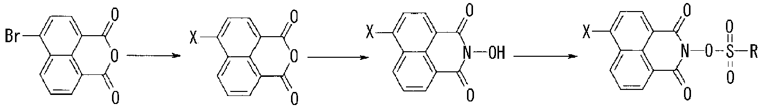 (wherein, X and R each represent the same group as in the Formula (I))

The sulfonic acid derivative compound of the present invention has a property of releasing a Lewis acid when irradiated with an active energy beam, such as a far-ultraviolet ray (e.g., EUV (Extreme Ultra-Violet), X-ray, F₂, ArF, KrF, i-line, h-line or g-line), an electron beam, a radiation or a high-frequency wave, and is thus capable of acting on an acid-reactive organic substance to cause decomposition and polymerization thereof. Therefore, the sulfonic acid derivative compound of the present invention is useful as a photoacid generator of positive and negative photoresists and as a cationic polymerization initiator used in a wide range of applications, including photoresists for preparation of lithographic plates and letterpress printing plates as well as printed circuit boards, ICs and LSIs; image formation such as relief image formation and image replication; and photo-curable inks, paints, adhesives and the like.

The sulfonic acid derivative compound of the present invention is used in a resist composition containing the above-described acid generator. Further, the sulfonic acid derivative compound of the present invention is useful for a cationically polymerizable composition containing the above-described cationic polymerization initiator.

When the sulfonic acid derivative compound of the present invention is used for an acid-reactive organic substance, the amount thereof to be used is not particularly restricted; however, it is used in a proportion of preferably 0.05 to 100 parts by mass, more preferably 0.05 to 20 parts by mass, with respect to 100 parts by mass of the acid-reactive organic substance. It is noted here that, depending on the factors such as the properties of the acid-reactive organic substance, the light irradiation intensity, the time required for reaction, the physical properties and the cost, the sulfonic acid derivative compound of the present invention may also be used in an amount that is greater or less than the above-described range.

In positive photoresists, a resin which changes toward having an increased solubility to a developing solution due to, for example, cleavage of a chemical bond of an ester group, an acetal group or the like caused by the action of an acid (hereinafter, such a resin is also referred to as "resist base resin") is used, whereas in negative photoresist resins, a compound or resin which changes to have a reduced solubility to a developing solution due to formation of a chemical bond, such as polymerization or cross-linking, caused by the action of an acid is used.

Examples of the resist base resin or compound include polyhydroxystyrenes and derivatives thereof; polyacrylic acids and derivatives thereof; polymethacrylic acids and derivatives thereof; copolymers formed by two or more selected from hydroxystyrene, acrylic acid, methacrylic acid and derivatives thereof; copolymers formed by two or more selected from hydroxystyrene, styrene and derivatives thereof; copolymers formed by three or more selected from cycloolefins and derivatives thereof, maleic anhydride, and acrylic acid and derivatives thereof; copolymers formed by three or more selected from cycloolefins and derivatives thereof, maleimide, and acrylic acid and derivatives thereof; polynorbornenes; high-molecular-weight polymers of one or more selected from the group consisting of metathesis ring-opening polymers; and these high-molecular-weight polymers which are partially substituted with an acid-labile group having an alkali dissolution-controlling ability. Examples of the acid-labile group incorporated into the high-molecular-weight polymers include tertiary alkyl groups, trialkylsilyl groups, oxoalkyl groups, aryl group-substituted alkyl groups, heteroalicyclic groups such as tetrahydropyran-2-yl group, tertiary alkylcarbonyl groups, tertiary alkylcarbonylalkyl groups, and alkyloxycarbonyl groups.

Detailed specific examples of the resist base resin or compound are disclosed in, for example, Japanese Unexamined Patent Application Publication No. 2003-192665, Claim 3 of Japanese Unexamined Patent Application Publication No. 2004-323704, and Japanese Unexamined Patent Application Publication No. H10-10733.

The polystyrene-equivalent weight-average molecular weight (Mw) of the resist base resin, which is determined by gel permeation chromatography (GPC), is usually 1,500 to 300,000, preferably 2,000 to 200,000, more preferably 3,000 to 100,000. In this case, when the Mw of the resist base resin is less than 1,500, the heat resistance as a resist tends to be reduced, whereas when the Mw is higher than 300,000,the developability and coatability as a resist tends to be impaired.

In the resist composition of the present invention, as long as the photoacid generator contains the sulfonic acid derivative compound of the present invention as an indispensable component, other photoacid generator may also be used as an optional component. From the standpoint of ensuring the sensitivity and developability as a resist, the photoacid generator is used in an amount of usually 0.01 to 20 parts by mass, preferably 0.5 to 10 parts by mass, with respect to 100 parts by mass of the resist base resin. In this case, when the amount of the photoacid generator is less than 0.01 parts by mass, the sensitivity and developability may be deteriorated, whereas when the amount is greater than 20 parts by mass, the transparency to radiation is reduced, which can makes it difficult to obtain a rectangular resist pattern.

When the sulfonic acid derivative compound of the present invention is used as a photoacid generator, other photoacid generator such as an iodonium salt compound or a sulfonium compound may also be used in combination. When other photoacid generator is used, it is preferably used in an amount of 10 to 200 parts by mass with respect to 100 parts by mass of the sulfonic acid derivative compound of the present invention.

In a photoresist in which the sulfonic acid derivative compound of the present invention is used as a photoacid generator, various additives may also be incorporated. Examples thereof include various resin additives, such as inorganic fillers, organic fillers, coloring agents (e.g., pigments and dyes), antifoaming agents, thickening agents, flame retardants, antioxidants, stabilizers and leveling agents. In the resist composition of the present invention, these various additives are used in an amount of preferably 50% by mass or less in total.

Before being used, the resist composition of the present invention is normally adjusted by being dissolved in a solvent to a total solid concentration of usually 5 to 50% by weight, preferably 10 to 25% by weight, and then being filtered through, for example, a filter having a pore size of about 0.2 µm. The resist composition of the present invention can be prepared by a method of, for example, mixing, dissolving or kneading a photoacid generator composed of the sulfonic acid derivative compound of the present invention, other photoacid generator(s), a resist base resin and other arbitrary component(s).

The resist composition of the invention is particularly useful as a chemically amplified resist. There are two types of chemically amplified resists: negative resists in which a chemical chain reaction is induced by the action of an acid generated from a photoacid generator on exposure and this causes a base resin to be cross-linked or change its polarity so as to make the resists insoluble in a developing solution; and positive resists which are made soluble in a developing solution by a change in the polarity induced by a deprotection reaction of a polymer side chain.

A light source used for the exposure of the photoresist is selected as appropriate from those emitting visible light, ultraviolet radiation, far-ultraviolet radiation, X-ray, charged particle beam or the like, in accordance with the type of the photoacid generator(s) used. The sulfonic acid derivative compound of the present invention can be suitably used in a resist which utilizes a variety of radiations, including far-ultraviolet radiations such as ArF excimer laser (wavelength: 193 nm) and KrF excimer laser (wavelength: 248 nm), X-rays such as synchrotron radiation, and charged particle beams such as electron beams and EUV.

When the sulfonic acid derivative compound of the present invention is used as a cationic polymerization initiator in a cationically polymerizable composition, in the cationically polymerizable composition, one or more cationically polymerizable compounds in which polymerization or cross-linking reaction is induced by the cationic polymerization initiator activated by irradiation with light are used in combination.

Representative examples of the cationically polymerizable compounds include epoxy compounds, oxetane compounds, cyclic lactone compounds, cyclic acetal compounds, cyclic thioether compounds, spiro-orthoester compounds and vinyl compounds, and one or more of these compounds can be used. Thereamong, epoxy compounds and oxetane compounds are suitable because of their availability and ease of handling.

As the epoxy compounds, for example, alicyclic epoxy compounds, aromatic epoxy compounds and aliphatic epoxy compounds are suitable.

Specific examples of the alicyclic epoxy compounds include polyglycidyl ethers of polyhydric alcohols having at least one alicyclic ring; and cyclohexene oxide or cyclopentene oxide-containing compounds obtained by epoxidizing a cyclohexene ring or cyclopentene ring-containing compound with an oxidizing agent. Examples of these compounds include hydrogenated bisphenol-A diglycidyl ether, 3,4-epoxycyclohexylmethyl-3,4-epoxycyclohexane carboxylate, 3,4-epoxy-1-methylcyclohexyl-3,4-epoxy-1-methylcyclohexane carboxylate, 6-methyl-3,4-epoxycyclohexylmethyl-6-methyl-3,4-epoxycyclohexane carboxylate, 3,4-epoxy-3-methylcyclohexylmethyl-3,4-epoxy-3-methylcyclohexane carboxylate, 3,4-epoxy-5-methylcyclohexylmethyl-3,4-epoxy-5-methylcyclohexane carboxylate, 2-(3,4-epoxycyclohexyl-5,5-spiro-3,4-epoxy)cyclohexane-metadioxane, bis(3,4-epoxycyclohexylmethyl)adipate, 3,4-epoxy-6-methylcyclohexyl carboxylate, methylenebis(3,4-epoxycyclohexane), dicyclopentadiene diepoxide, ethylenebis(3,4-epoxycyclohexanecarboxylate), dioctyl epoxyhexahydrophthalate and di-2-ethylhexyl epoxyhexahydrophthalate.

Examples of commercially available products that can be suitably used as the alicyclic epoxy compounds include UVR-6100, UVR-6105, UVR-6110, UVR-6128, and UVR-6200 (all of which are manufactured by Union Carbide Corporation); Celloxide 2021, Celloxide 2021P, Celloxide 2081, Celloxide 2083, Celloxide 2085, Celloxide 2000, Celloxide 3000, Cyclomer A200, Cyclomer M100, Cyclomer M101, Epolead GT-301, Epolead GT-302, Epolead 401, Epolead 403, ETHB, and Epolead HD300 (all of which are manufactured by Daicel Chemical Industries, Ltd.); and KRM-2110 and KRM-2199 (both of which are manufactured by ADEKA Corporation).

Among these alicyclic epoxy compounds, epoxy resins having a cyclohexene oxide structure are preferred because of their curability (curing rate).

Further, specific examples of the aromatic epoxy compounds include polyglycidyl ethers of polyhydric phenols having at least one aromatic ring or alkylene oxide adducts thereof, such as glycidyl ethers of bisphenol A, bisphenol F or an alkylene oxide adduct thereof; and epoxy novolac resins.

Specific examples of the aliphatic epoxy compounds include polyglycidyl ethers of aliphatic polyhydric alcohols or alkylene oxide adducts thereof; polyglycidyl esters of aliphatic long-chain polybasic acids; homopolymers synthesized by vinyl polymerization of glycidyl acrylate or glycidyl methacrylate; and copolymers synthesized by vinyl polymerization of glycidyl acrylate or glycidyl methacrylate and other vinyl monomer(s). Representative examples of these compounds include glycidyl ethers of polyhydric alcohols, such as 1,4-butanediol diglycidyl ether, 1,6-hexanediol diglycidyl ether, glycerol triglycidyl ether, trimethylolpropane triglycidyl ether, sorbitol tetraglycidyl ether, dipentaerythritol hexaglycidyl ether, polyethylene glycol diglycidyl ether and polypropylene glycol diglycidyl ether; polyglycidyl ethers of polyether polyols obtained by addition of one or more alkylene oxides to an aliphatic polyhydric alcohol, such as propylene glycol, trimethylolpropane and glycerin; and diglycidyl esters of aliphatic long-chain dibasic acids. Examples thereof also include monoglycidyl ethers of aliphatic higher alcohols; monoglycidyl ethers of phenol, cresol, butylphenol, or polyether alcohols obtained by addition of an alkylene oxide thereto; glycidyl esters of higher fatty acids; epoxidized soybean oil; octyl epoxystearate; butyl epoxystearate; and epoxidized polybutadienes.

Examples of commercially available products that can be suitably used as the aromatic or aliphatic epoxy compounds include Epikote 801 and Epikote 828 (both of which are manufactured by Yuka Shell Epoxy K.K.); PY-306, 0163, and DY-022 (all of which are manufactured by Ciba Specialty Chemicals K.K.); KRM-2720, EP-4100, EP-4000, EP-4080, EP-4900, ED-505 and ED-506 (all of which are manufactured by ADEKA Corporation); Epolight M-1230, Epolight EHDG-L, Epolight 40E, Epolight 100E, Epolight 200E, Epolight 400E, Epolight 70P, Epolight 200P, Epolight 400P, Epolight 1500NP, Epolight 1600, Epolight 80MF, Epolight 100MF, Epolight 4000, Epolight 3002 and Epolight FR-1500 (all of which are manufactured by Kyoeisha Chemical Co., Ltd.); and Suntohto ST0000, YD-716, YH-300, PG-202, PG-207, YD-172 and YDPN638 (all of which are manufactured by Tohto Kasei Co., Ltd.).

Further, specific examples of the oxetane compounds include 3-ethyl-3-hydroxymethyloxetane, 3-(meth)allyloxymethyl-3-ethyloxetane, (3-ethyl-3-oxetanylmethoxy)methylbenzene, 4-fluoro-[1-(3-ethyl-3-oxetanylmethoxy)methyl]benzene, 4-methoxy-[1-(3-ethyl-3-oxetanylmethoxy)methyl]benzene, [1-(3-ethyl-3-oxetanylmethoxy)ethyl]phenyl ether, isobutoxymethyl(3-ethyl-3-oxetanylmethyl)ether, isobornyloxyethyl(3-ethyl-3-oxetanylmethyl)ether, isobornyl(3-ethyl-3-oxetanylmethyl)ether, 2-ethylhexyl(3-ethyl-3-oxetanylmethyl)ether, ethyl diethylene glycol (3-ethyl-3-oxetanylmethyl)ether, dicyclopentadiene(3-ethyl-3-oxetanylmethyl)ether, dicyclopentenyloxyethyl(3-ethyl-3-oxetanylmethyl)ether, dicyclopentenyl(3-ethyl-3-oxetanylmethyl)ether, tetrahydrofurfuryl(3-ethyl-3-oxetanylmethyl)ether, tetrabromophenyl(3-ethyl-3-oxetanylmethyl)ether, 2-tetrabromophenoxyethyl(3-ethyl-3-oxetanylmethyl)ether, tribromophenyl(3-ethyl-3-oxetanylmethyl)ether, 2-tribromophenoxyethyl(3-ethyl-3-oxetanylmethyl)ether, 2-hydroxyethyl(3-ethyl-3-oxetanylmethyl)ether, 2-hydroxypropyl(3-ethyl-3-oxetanylmethyl)ether, butoxyethyl(3-ethyl-3-oxetanylmethyl)ether, pentachlorophenyl(3-ethyl-3-oxetanylmethyl)ether, pentabromophenyl(3-ethyl-3-oxetanylmethyl)ether, bornyl(3-ethyl-3-oxetanylmethyl)ether, 3,7-bis(3-oxetanyl)-5-oxa-nonane, 3,3'-(1,3-(2-methylenyl)propanediyl-bis(oxymethylene))bis-(3-ethyloxetane), 1,4-bis[(3-ethyl-3-oxetanylmethoxy)methyl]benzene, 1,2-bis[(3-ethyl-3-oxetanylmethoxy)methyl]ethane, 1,3-bis[(3-ethyl-3-oxetanylmethoxy)methyl]propane, ethylene glycol-bis(3-ethyl-3-oxetanylmethyl)ether, dicyclopentenyl-bis(3-ethyl-3-oxetanylmethyl)ether, triethylene glycol-bis(3-ethyl-3-oxetanylmethyl)ether, tetraethylene glycol-bis(3-ethyl-3-oxetanylmethyl)ether, tricyclodecanediyldimethylene(3-ethyl-3-oxetanylmethyl)ether, trimethylolpropane tris(3-ethyl-3-oxetanylmethyl)ether, 1,4-bis(3-ethyl-3-oxetanylmethoxy)butane, 1,6-bis(3-ethyl-3-oxetanylmethoxy)hexane, pentaerythritol tris(3-ethyl-3-oxetanylmethyl)ether, pentaerythritol tetrakis(3-ethyl-3-oxetanylmethyl)ether, polyethylene glycol-bis(3-ethyl-3-oxetanylmethyl)ether, dipentaerythritol hexakis(3-ethyl-3-oxetanylmethyl)ether, dipentaerythritol pentakis(3-ethyl-3-oxetanylmethyl)ether, dipentaerythritol tetrakis(3-ethyl-3-oxetanylmethyl)ether, caprolactone-modified dipentaerythritol hexakis(3-ethyl-3-oxetanylmethyl)ether, caprolactone-modified dipentaerythritol pentakis(3-ethyl-3-oxetanylmethyl)ether, ditrimethylolpropane tetrakis(3-ethyl-3-oxetanylmethyl)ether, EO-modified bisphenol-A bis(3-ethyl-3-oxetanylmethyl)ether, PO-modified bisphenol-A bis(3-ethyl-3-oxetanylmethyl)ether, EO-modified hydrogenated bisphenol-A bis(3-ethyl-3-oxetanylmethyl)ether, PO-modified hydrogenated bisphenol-A bis(3-ethyl-3-oxetanylmethyl)ether, and EO-modified bisphenol-F (3-ethyl-3-oxetanylmethyl)ether.

The use of these oxetane compounds is effective and thus preferred particularly when flexibility is required.

Specific examples of other compounds used as the cationically polymerizable compounds include cyclic lactone compounds such as β-propiolactone and ε-caprolactone; cyclic acetal compounds such as trioxane, 1,3-dioxolane and 1,3,6-trioxanecyclooctane; cyclic thioether compounds such as tetrahydrothiophene derivatives; spiro-orthoester compounds obtained by reaction between any of the above-described epoxy compounds and lactone; vinyl ether compounds such as ethylene glycol divinyl ether, alkyl vinyl ether, 2-chloroethyl vinyl ether, 2-hydroxyethyl vinyl ether, triethylene glycol divinyl ether, 1,4-cyclohexanedimethanol divinyl ether, hydroxybutyl vinyl ether, and the propenyl ether of propylene glycol; vinyl compounds such as ethylenically unsaturated compounds, including styrene, vinylcyclohexene, isobutylene and polybutadiene; oxolane compounds such as tetrahydrofuran and 2,3-dimethyltetrahydrofuran; thiirane compounds such as ethylene sulfide and thioepichlorohydrin; thietane compounds such as 1,3-propyne sulfide and 3,3-dimethylthietane; and silicones, all of which compounds are well-known.

When the sulfonic acid derivative compound of the present invention is used as a cationic polymerization initiator, it is used in an amount of preferably 0.01 parts by mass to 10 parts by mass, more preferably 0.1 parts by mass to 5 parts by mass, with respect to 100 parts by mass of the cationically polymerizable compound. When this amount is less than 0.01 parts by mass, curing may be insufficient, whereas when the amount is greater than 10 parts by mass, not only an increase in the effect of the use cannot be attained but also the sulfonic acid derivative compound may adversely affect the physical properties of the resulting cured article.

Further, the sulfonic acid derivative compound of the present invention is blended with the above-described cationically polymerizable compound and other various additives and used as a cationically polymerizable composition. Examples of the various additives include organic solvents; benzotriazole-based, triazine-based and benzoate-based ultraviolet absorbers; phenolic, phosphorus-based and sulfur-based antioxidants; antistatic agents, such as cationic surfactants, anionic surfactants, nonionic surfactants and amphoteric surfactants; flame retardants such as halogen-containing compounds, phosphate compounds, phosphoric amide compounds, melamine compounds, fluorocarbon resins, metal oxides, melamine (poly)phosphate and piperazine (poly)phosphate; hydrocarbon-based, fatty acid-based, aliphatic alcohol-based, aliphatic ester-based, aliphatic amide-based or metal soap-based lubricants; coloring agents such as dyes, pigments and carbon black; silicate-based inorganic additives, such as fumed silica, microparticulate silica, silica rock, diatomaceous earth, clay, kaolin, silica gel, calcium silicate, sericite, kaolinite, flint, feldspar powder, vermiculite, attapulgite, talc, mica, minnesotaite, pyrophyllite and silica; fillers such as glass fibers and calcium carbonate; crystallization agents such as nucleating agents and crystallization-promoting agents; silane coupling agents; rubber elasticity-imparting agents such as flexible polymers; sensitizers; basic compounds; photoradical initiators; thermal cationic initiators; and cross-linking agents. In the cationically polymerizable composition of the present invention, these various additives are used in a total amount of 50% by mass or less.

Further, in order to facilitate dissolution of the sulfonic acid derivative compound of the present invention into the cationically polymerizable compound, the sulfonic acid derivative compound of the present invention, before being used, can be dissolved in an appropriate solvent in advance (e.g., propylene carbonate, carbitol, carbitol acetate, butyrolactone or propylene glycol-1-monomethyl ether-2-acetate).

By irradiating the cationically polymerizable composition with an energy beam such as ultraviolet radiation, the cationically polymerizable composition can be cured to a dry-to-touch state or solvent-insoluble state usually 0.1 second to several minutes thereafter. As an appropriate energy beam, any energy beam may be used as long as it induces decomposition of the cationic polymerization initiator; however, it is preferred to use an electromagnetic energy beam having a wavelength of 2,000 Å to 7,000 Å that is emitted from, for example, an ultrahigh, high, medium or low-pressure mercury lamp, a xenon lamp, a carbon arc lamp, a metal halide lamp, a fluorescent lamp, a tungsten lamp, an excimer lamp, a germicidal lamp, an excimer laser, a nitrogen laser, an argon ion laser, a helium-cadmium laser, a helium neon laser, a krypton ion laser, a semiconductor laser, a YAG laser, a light-emitting diode or a CRT light source; or a high-energy beam such as an electron beam, X-ray or radiation.

The time of exposure to an energy beam is variable depending on the intensity of the energy beam, the coating film thickness and the cationically polymerizable compound; however, an exposure of 0.1 second to 10 seconds or so is usually sufficient. Still, a longer exposure time is preferred for a relatively thick coating material. In 0.1 second to several minutes after the exposure to an energy beam, most of the composition becomes dry-to-touch as a result of cationic polymerization and, depending on the case, it is also preferred to use thermal energy provided by heating, a thermal head or the like in combination so as to accelerate the cationic polymerization.

Specific examples of applications in which the resist composition and cationically polymerizable composition of the present invention can be used include, but not particularly limited to: optical filters; paints; coating agents; lining agents; adhesives; printing plates; insulating varnishes; insulation sheets; laminated plates; printed circuit boards; sealants for semiconductor devices, LED packages, liquid crystal inlets, organic EL devices, optical elements, electrical insulating materials, electronic components, separation membranes and the like; molded materials; putties; glass fiber impregnants; fillers; passivation films for semiconductors, solar cells and the like; interlayer insulation films and surface protection films that are used in thin-film transistors (TFT), liquid crystal displays, organic EL displays, printed circuit boards and the like; color filters of color televisions, PC monitors, personal digital assistants and CCD image sensors; electrode materials for plasma display panels; printing inks; dental compositions; resins for stereolithography; liquid-form films and dry films; micromachine components; glass fiber cable coatings; materials for holographic recording; magnetic recording materials; optical switches; plating masks; etching masks; screen printing stencils; touch panels such as transparent conductive films; MEMS elements; nanoimprint materials; photofabrication applications such as two-dimensional and three-dimensional high-density mounting and the like of semiconductor packages; decoration sheets; artificial nails; glass-alternative optical films; electronic papers; optical disks; micro-lens arrays used in projectors, optical communication lasers and the like; prism lens sheets used in backlights of liquid crystal displays; Fresnel lens sheets used in the screens of projection televisions and the like; lens parts of lens sheets such as lenticular lens sheets; backlights and the like using these sheets; optical lenses such as microlenses and image pickup lenses; optical elements; optical connectors; optical waveguides; insulation packings; heat-shrinkable rubber tubes; O-rings; sealing agents for display devices; protective materials; optical fiber protection materials; adhesives; die bonding agents; high-heat radiation materials; high-heat resistant sealing materials; members for solar cells, fuel cells and secondary batteries; solid electrolytes for batteries; insulation coating materials; heat-sensitive drums for copying machines; gas separation membranes; civil engineering and construction materials, such as concrete protecting materials, linings, soil injection agents, sealing agents, cold-heat storage materials, glass coatings and foams; medical materials such as tube/seal materials, coating materials, sealing materials for sterilizers, contact lenses, oxygen enrichment membranes and biochips; automobile components; and various mechanical components.

### EXAMPLES

The present invention will now be further described by way of examples, comparative examples and evaluation examples thereof. However, the present invention is not restricted thereto by any means.

### [Example 1] Production of Compound No. 4

Under a nitrogen atmosphere, while stirring a solution containing 500 ml of tetrahydrofuran cooled to -70°C and 74.3 mmol of 1-butyllithium, a suspension of 750 mmol of zinc bromide and 600 ml of tetrahydrofuran was added thereto dropwise at a rate which did not increase the temperature of the reaction system above -55°C. Then, the temperature of the reaction system was restored to 15°C, and the reaction system was stirred for 1 hour to prepare a butylzinc reagent.

Under a nitrogen atmosphere, the thus obtained butylzinc reagent was added dropwise to a mixed solution of 300 mmol of 4-bromonaphthalic anhydride, 6.00 mmol of bis(diphenylphosphino)palladium dichloride (PdCl₂(dppf)₂) and 500 ml of tetrahydrofuran, and the resultant was stirred at room temperature for 1 hour. Subsequently, 1,000 ml of water was added thereto and an organic phase was obtained by oil-water separation. To a solid phase obtained by concentrating the organic phase, 500 ml of toluene and 90.0 g of silica gel were added and stirred, and a solid phase was then separated by filtration. To a solid phase obtained by concentrating the resulting filtrate, 350 ml of methanol was added, and the resulting solution was heated and filtered to obtain a filtrate, which was then cooled and allowed to crystallize. The resulting crystals were recovered by filtration and washed with isopropyl alcohol. Thereafter, the crystals were vacuum-dried at 45°C to obtain 26.5 g of pale-yellow crystals (4-butylnaphthalic anhydride).

The thus obtained 4-butylnaphthalic anhydride in an amount of 20.0 mmol was suspended in 30 g of dimethylformamide, and 24.0 mmol of NH₂OH-HCl was added thereto at room temperature. Then, 2.00 g of 48% aqueous sodium hydroxide solution was added thereto dropwise, and the resultant was stirred for 3 hours. Further, 20.0 g of water and 0.30 g of 35% hydrochloric acid were added thereto, and the resultant was stirred for another one hour. The resulting precipitates were recovered by filtration, washed with a mixture of methanol and water, and then vacuum-dried at 45°C to obtain 5.06 g of hydroxyimide.

To a mixture of 10.0 mmol of the thus obtained hydroxyimide and 18.9 g of chloroform, 15.9 mmol of pyridine was added and, while maintaining the temperature of the resultant to be 2°C or lower, 13.2 mmol of trifluoromethanesulfonic anhydride was further added and stirred at room temperature for 1 hour. To the resulting reaction solution, 20 g of water was added, and an oil phase obtained by oil-water separation was washed twice with 0.5% aqueous sodium hydroxide solution, once with 3% hydrochloric acid, and then five times with water. A solid phase obtained by concentrating the organic phase was heat-dissolved in chloroform, and the resultant was filtered to obtain a filtrate, which was then allowed to crystallize by adding thereto methanol. The resulting crystals were recovered by filtration and vacuum-dried at 45°C to obtain 3.06 g of pale-yellow crystals. The thus obtained crystals were subjected to various analyses and it was confirmed that the crystals were Compound No. 4. The analysis results are shown in Tables 1 to 3.

### [Example 2] Production of Compound No. 5

White crystals were obtained in an amount of 3.00 g by the same formulations and procedures as in Example 1, except that nonafluorobutanesulfonic anhydride was used in place of trifluoromethanesulfonic anhydride. The thus obtained crystals were subjected to various analyses and it was confirmed that the crystals were Compound No. 5. The analysis results are shown in Tables 1 to 3.

### [Example 3] Production of Compound No. 7

To a mixture of 10.0 mmol of the hydroxyimide obtained in Example 1 and 22.0 g of chloroform, 13.6 mmol of triethylamine was added and, while maintaining the temperature of the resultant to be 2°C or lower, a solution of 12.0 mmol of 2,4,6-triisopropylbenzene sulfonic acid chloride and 7.3 g of chloroform was further added dropwise. After stirring the resultant at room temperature for 5 hours, 13.0 g of water was added to the resulting reaction solution, and an oil phase obtained by oil-water separation was washed twice with 0.5% aqueous sodium hydroxide solution, once with 3% hydrochloric acid, and then five times with water. A solid phase obtained by concentrating the organic phase was heat-dissolved in methanol, and the resultant was allowed to cool. Then, precipitated crystals were recovered by filtration and heat-dissolved in chloroform, and the resultant was filtered to obtain a filtrate, which was subsequently allowed to crystallize by adding thereto methanol. The resulting crystals were recovered by filtration and vacuum-dried at 45°C to obtain 4.37 g of white crystals. The thus obtained crystals were subjected to various analyses and it was confirmed that the crystals were Compound No. 7. The analysis results are shown in Tables 1 to 3.

### [Example 4] Production of Compound No. 10

White crystals were obtained in an amount of 3.00 g by the same formulations and procedures as in Example 2, except that d-camphorsulfonic acid chloride was used in place of 2,4,6-triisopropylbenzene sulfonic acid chloride. The thus obtained crystals were subjected to various analyses and it was confirmed that the crystals were Compound No. 10. The analysis results are shown in Tables 1 to 3.

### [Example 5] Production of Comparative Compound No. 20

Pale-yellow crystals were obtained in an amount of 2.74 g by the same formulations and operations as in Example 1, except that ethyllithium was used in place of 1-butyllithium. The thus obtained crystals were subjected to various analyses and it was confirmed that the crystals were Compound No. 20. The analysis results are shown in Tables 1 to 3.

### [Example 6] Production of Compound No. 6

Pale-yellow crystals were obtained in an amount of 3.56 g by the same formulations and operations as in Example 3, except that p-toluenesulfonic acid chloride was used in place of 2,4,6-triisopropylbenzene sulfonic acid chloride. The thus obtained crystals were subjected to various analyses and it was confirmed that the crystals were Compound No. 6. The analysis results are shown in Tables 1 to 3.

### [Example 7] Production of Compound No. 35

White solids were obtained in an amount of 3.70 g by the same formulations and operations as in Example 3, except that 4-trifluoromethylbenzenesulfonic acid chloride was used in place of 2,4,6-triisopropylbenzene sulfonic acid chloride. The thus obtained crystals were subjected to various analyses and it was confirmed that the crystals were Compound No. 35. The analysis results are shown in Tables 1 to 3.

### [Example 8] Production of Compound No. 38

Pale-yellow solids were obtained in an amount of 3.05 g by the same formulations and operations as in Examples 1 and 3, except that 2-ethylhexyllithium was used in place of 1-butyllithium. The thus obtained crystals were subjected to various analyses and it was confirmed that the crystals were Compound No. 38. The analysis results are shown in Tables 1 to 3.

### [Example 9] Production of Comparative Compound No. 40

Pale-yellow solids were obtained in an amount of 2.12 g by the same formulations and operations as in Examples 1 and 3, except that zinc tetradecylbromide was used in place of 1-butyllithium. The thus obtained crystals were subjected to various analyses and it was confirmed that the crystals were Compound No. 40. The analysis results are shown in Tables 1 to 3.

**[Table 1]**

| | Chemical shift/ppm (multiplicity, proton number) J: coupling constant |
|---|---|
| Compound No. 4 (Acetonitirle-d3) | 8.65-8.58(m,2H), 8.52(d,1H,J=7.3 Hz), 7.86(dd,1H,J=7,3,7.3 Hz), 7.71(d,1H,J=7.9 Hz), 3.21(t,2H,J=7.9 Hz), 1.76-1.68(m,2H), 1.51-1.40(m,2H), 0.96(t,3H,J=7.3 Hz). |
| Compound No. 5 (Acetonitirle-d3) | 8.61-8.56(m,2H), 8.49(d,1H,J=7.9 Hz), 7.83(t,1H,J=7.9 Hz), 7.68(d,1H,J=7.3 Hz), 3.18(t,2H,J=7.6 Hz), 1.74-1.65(m,2H), 1.50-1.39(m,2H), 0.95(t,3H,J =7.3 Hz). |
| Compound No. 7 (Acetonitirle-d3) | 8.57(d,1H,J=8.5 Hz), 8.49(d,1H,J=7.3 Hz), 8.40(d,1H,J=7.9 Hz), 7.81(dd,1H,J=7.9,7.3 Hz), 7.66(d,1H,J=7.3 Hz), 7.35(s,2H), 3.99(sept,2H,J=6.7 Hz), 3.19(t,2H,J=7.9 Hz), 3.00(sept,1H,J=6.7 Hz), 1.75-1.65(m,2H), 1.50-1.48(m,2H), 1.28(d,6H,J=6.7 Hz), 1.22(d,12H,J=6.7 Hz),0.95 (t,3H,J=7.3 Hz) |
| Compound No. 10 (Acetonitirle-d3) | 8.65(d,2H,J=7.9 Hz), 8.47(d,1H,J=7.9 Hz), 7.82(dd,1H,J=7.9,7.9 Hz), 7.67(d,1H,J=7.3 Hz), 4.11(d,2H,J=15.2 Hz), 3.95(d,1H,J=14.6 Hz), 3.18(t,2H,J=7.3 Hz), 2.45-2.30(m,2H), 2.17-1.90(m,4H), 1.80-1.67(m,3H), 1.51-1.40(m,3H), 0.96(t,3H,J=7.3 Hz), 0.91(s,3H) |
| Comparative Compound No. 20 (Acetonitirle-d3) | 8.65-8.58(m,2H), 8.52(d,1H,J=7.3 Hz), 7.86(dd,1H,J=7.3,7.3 Hz),7.71(d,1H,J=7.9 Hz), 3.21(t,2H,J=7.9 Hz), 1.76-1.68(m,2H), 1.51-1.40(m,2H), 0.96(t,3H,J=7.3 Hz) |
| Compound No. 6 (DMSO-*d₆*) | 8.63(d, 1H,*J*=8.5 Hz), 8.46(d,1H,*J*=7.3 Hz), 8.37(d,1H,*J*=7.3 Hz), 7.95-7.75 (m,3H), 7.71(d,1H,*J*=7.9 Hz), 7.50(d,2H,*J*=7.9 Hz), 3.18(d,2H,*J*=7.6 Hz), 2.45(s,3H), 1.70-1.60(m,2H), 1.45-1.32(m,2H), 0.91(t,3H,*J*=7.3 Hz). |
| Compound No. 35 (CDCl₃) | 8.59(d,1H,*J*=8.5 Hz), 8.48(d,1H,*J*=6.7 Hz), 8.39(d,1H,*J*=7.3 Hz), 8.22(d,2H,*J*=8.5 Hz), 7.96(d,2H,*J*=8.5 Hz), 7.82(dd,1H,*J*=8.5,7.3 Hz), 7.67(d,1H,*J*=7.9 Hz), 3.20(t,2H,*J*=7.9 Hz), 1.76-1.67(m,2H), 1.55-1.39(m,2H), 0.96(t,3H,*J*=7.3 Hz). |
| Compound No. 38 (CDCl₃) | 8.64(dd,1H,*J*=7.3,1.2 Hz), 8.54(d,1H,*J*=7.3 Hz), 8.46(d,1H,*J*=8.5 Hz), 8.05(d,2H,*J*=8.5 Hz), 7.79(dd,1H,*J*=8.5,7.3 Hz), 7.59(d,1H,*J*=7.3 Hz), 7.43(d,2H,*J*=7.9 Hz), 3.10(d,2H,*J*=7.3 Hz), 2.50(s,3H), 1.82-1.70(m,1H), 1.45-1.21(m,8H), 0.92(t,3H,*J*=7.3 Hz), 0.87(t,3H, *J*=7.3 Hz). |
| Comparative Compound No. 40(CDCl₃) | 8.64(d,1H,*J*=6.7 Hz), 8.55(d,1H,*J*=7.3 Hz), 8.47(d,1H,*J*=8.5 Hz), 8.05(d,2H,*J*=8.5 Hz), 7.80(dd,1H,*J*=7.9,7.9 Hz), 7.62(d,1H,*J*=7.9 Hz), 7.43(d,2H,*J*=7.9 Hz), 3.18(t,2H,*J*=7.6 Hz), 2.50(s,3H), 1.82-1.72(m,2H), 1.49-1.20(m,22H), 0.88(t,3H,*J*=6.7 Hz). |

**[Table 2]**

| | IR absorption spectrum/cm⁻¹ |
|---|---|
| Compound No. 4 | 2969, 2938, 2883, 1716, 1579, 1434, 1397, 1321, 1228, 1174, 1130, 953, 853 |
| Compound No. 5 | 2970, 2940, 2878, 1730, 1703, 1582, 1436, 1350, 1325, 1214, 1196, 1142, 1018, 953, 894, 851 |
| Compound No. 7 | 2958, 2932, 2870, 1732, 1702, 1589, 1388, 1382, 1230, 1186, 1020, 895 |
| Compound No. 10 | 2956, 2929, 2873, 1730, 1701, 1587, 1389, 1331, 1227, 1192, 1180, 1089, 1019, 954, 892, 812 |
| Comparative Compound No. 20 | 2969, 2938, 2883, 1716, 1579, 1434, 1397, 1321, 1228, 1174, 1130, 953, 853 |
| Compound No. 6 | 2954, 1729, 1696, 1583, 1384, 1335, 1226, 1180, 1015, 948, 894, 813, 772, 705, 694, 666, 647, 564, 557, 532 |
| Compound No. 35 | 2956, 1730, 1692, 1584, 1404, 1395, 1314, 1175, 1139, 1108, 1059, 1013, 893, 852, 772, 719, 687, 659, 607 |
| Compound No. 38 | 2918, 2869, 1725, 1701, 1586, 1366, 1327, 1178, 1090, 954, 893, 814, 777, 710, 702, 684 |
| Comparative Compound No. 40 | 2918, 2849, 1729, 1695, 1584, 1386, 1226, 1181, 1019, 948, 894, 812, 772, 704, 693, 647 |

**[Table 3]**

| | Melting point (°C) | Weight loss starting point (°C) |
|---|---|---|
| Compound No. 4 | 110 | 250 |
| Compound No. 5 | 106 | 242 |
| Compound No. 7 | 131 | 267 |
| Compound No. 10 | 134 | 279 |
| Comparative Compound No. 20 | 145 | 253 |
| Compound No. 6 | 145.1 | 314.8 |
| Compound No. 35 | 192 | 302 |
| Compound No. 38 | 129.6 | 304.9 |
| Comparative Compound No. 40 | 95.5 | 301.8 |

### [Example 10] Preparation and Evaluation of Negative Resist Composition

A hexafluoroantimonate of Compound No. 5 was synthesized separately. A resin solution was prepared by dissolving 100 g of EPPN-201 manufactured by Nippon Kayaku Co., Ltd. in 100 g of methyl ethyl ketone (MEK), and 0.05 g of the hexafluoroantimonate was dissolved in 8.00 g of this resin solution to prepare a resist solution. The thus obtained resist solution was coated on an aluminum plate using a #9 bar coater and dried at 80°C for 10 minutes, followed by exposure to light using an ultrahigh-pressure mercury lamp and an irradiation spectroscope. The resultant was baked at 80°C for 10 minutes, developed by immersion in MEK for 30 seconds, and then washed with xylene. The resulting coating film was cured by irradiation with light having a wavelength of 405 nm.

Thereafter, the coating film was left to stand at room temperature for 24 hours. The coating film was not damaged even when it was rubbed back and forth 200 times with a cotton swab soaked in methyl ethyl ketone; therefore, it was confirmed that the curing progressed sufficiently.

### [Example 11] Production and Evaluation of Cationically Polymerizable Composition

To a mixture of 80 g of 3,4-epoxycyclohexylmethyl-3,4-epoxycyclohexyl carboxylate and 20 g of 1,4-butanediol diglycidyl ether, 4 mmol of Compound No. 5 was added, and the resulting solution was thoroughly stirred to be homogenized. This solution was coated on an aluminum-coated paper using a #3 bar coater and then irradiated with light of an 80 W/cm high-pressure mercury lamp using a belt conveyor-equipped irradiation apparatus. The distance between the lamp and the belt conveyor was 10 cm, and the line speed of the belt conveyor was 8 m/min.

After the solution was cured, the resulting coating film was left to stand at room temperature for 24 hours. The coating film was not damaged even when it was rubbed back and forth 200 times with a cotton swab soaked in methyl ethyl ketone; therefore, it was confirmed that the curing progressed sufficiently.

### [Evaluation Example 1] Evaluation of UV Absorption Spectrum and Solubility

Compound Nos. 4, 5, 7, 10, 6, 35 and 38 and Comparative Compounds 1 represented by the Formula below and Comparative Compounds 20 and 40 were each dissolved in acetonitrile, and their absorption spectra were measured using a solar photometer U-3010 to determine the values of λmax and ε (molar extinction coefficient) for absorption in a range of 300 nm to 400 nm as well as the value of ε at 365 nm. In addition, their solubility to propylene glycol monomethyl ether acetate at 25°C was evaluated. As for the solubility, the concentration of each compound was increased by 1%-by-mass increments and the concentration at which no compound was left undissolved was determined and, in the results shown in Table 4, the solubility is indicated in terms of this concentration.

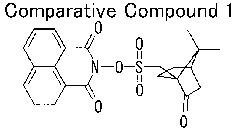

**[Table 4]**

| | Maximum absorption wavelength (nm) ε | ε at 365nm | Solubility (% by mass) |
|---|---|---|---|
| Compound No. 4 | 346 | 1.16 × 10⁴ | 20 |
| | 1.65 × 10⁴ | | |
| Compound No. 5 | 345 | 1.11 × 10⁴ | 8 |
| | 1.60 × 10⁴ | | |
| Compound No. 7 | 344 | 1.06 × 10⁴ | 5 |
| | 1.65 × 10⁴ | | |
| Compound No. 10 | 346 | 1.17 × 10⁴ | 5 |
| | 1.68 × 10⁴ | | |
| Comparative Compound No. 20 | 344 | 9.95 × 10³ | 5 |
| | 1.64 × 10⁴ | | |
| Compound No. 6 | 343.5 | 1.02 × 10⁴ | 2.3 |
| | 1.67 × 10⁴ | | |
| Compound No. 35 | 345.0 | 1.11 × 10⁴ | 2.0 |
| | 1.68 × 10⁴ | | |
| Compound No. 38 | 345 | 1.24 × 10⁴ | 1.6 |
| | 1.76 × 10⁴ | | |
| Comparative Compound No. 40 | 344.5 | 1.05 × 10⁴ | 1.9 |
| | 1.68 × 10⁴ | 10⁴ | |
| Comparative Compound 1 | 335 | 6.48 × 10² | 2 |
| | 1.34 × 10⁴ | | |

As seen from Table 4, comparing the compounds of the present invention and Comparative Compound 1, all of the compounds of the present invention showed greater absorption for light having a wavelength of 365 nm. In addition, Compound Nos. 4 and 5 both had a high solubility. Moreover, among Compound Nos. 4, 5, 7 and 10 whose sulfonate moiety is trifluoromethane sulfonate, Compound No. 4 had the highest solubility and, among Compound Nos. 6, 38 and 40 whose sulfonate moiety is toluene sulfonate, Compound No. 6 had the highest solubility. Between Compound Nos. 4 and 20 that have different Xs in the Formula (I), Compound No. 4 had a higher solubility.

### [Evaluation Example 2] Acid Generation Rate

For each of Compound Nos. 4, 7, 10 and 20 as well as Comparative Compound 1, a 0.5%-by-mass acetonitrile/water mixed solution (volume ratio: acetonitrile/water = 9/1) was prepared, and 5.0 g thereof was placed in a Petri dish having an inner diameter of 50 mm. Using a UV lamp and a cut filter transmitting only light having a wavelength of about 365 nm, which are manufactured by Hoya Candeo Optronics Corporation, the mixed solution was irradiated with UV having an intensity of 100 mW/cm². The irradiation time was 10 seconds, 30 seconds and 60 seconds for each solution. After the irradiation, the resultant was diluted with 45 g of acetonitrile/water mixed solution (volume ratio: acetonitrile/water = 9/1) and then titrated with 0.1 N aqueous potassium hydroxide solution using an automatic titrator manufactured by Hiranuma Sangyo Co., Ltd. (COM-1600). The acid concentration was determined from the used volume and the acid generation rate (mol %) was calculated therefrom. The results thereof are shown in Table 5.

**[Table 5]**

| | 10 seconds | 30 seconds | 60 seconds |
|---|---|---|---|
| Compound No. 4 | 28.3 | 57.4 | 59.0 |
| Compound No. 7 | 0.4 | 1.1 | 1.6 |
| Compound No. 10 | - | 0.3 | 0.9 |
| Comparative Compound No. 20 | 29.1 | 47.7 | 65.5 |
| Compound No. 6 | 18.1 | 18.3 | 20.1 |
| Compound No. 35 | 4.1 | 6.8 | 11.1 |
| Compound No. 38 | 13.4 | 14.3 | 16.4 |
| Comparative Compound No. 40 | 9.9 | 17.4 | 18.3 |
| Comparative Compound 1 | - | 2.2 | 6.6 |

As for Compound No. 10, the amount of acid generation obtained by 10-second irradiation was so small that the measurement could not be performed.

From Table 5, comparing Compound Nos. 4 and 20 whose sulfonate moiety is trifluoromethane sulfonate with Comparative Compound 1, it was confirmed that Compound Nos. 4 and 20 according to the present invention have superior acid generation capacity. Furthermore, comparing Compound Nos. 4 and 20 with Compound No 7 and Compound No. 10, which have different sulfonate moieties, it was confirmed that Compound Nos. 4 and 20 shows higher acid generation rates.

## Claims

1. A sulfonic acid derivative compound, which is represented by the following Formula (I): 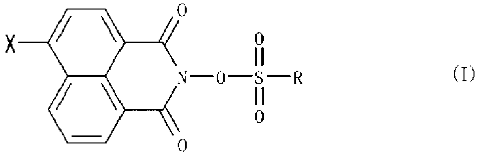 (wherein, X represents a linear or branched alkyl group having 3 to 8 carbon atoms; and R represents an aliphatic hydrocarbon group having 1 to 18 carbon atoms, an aryl group having 6 to 20 carbon atoms, an arylalkyl group having 7 to 20 carbon atoms, an aryl group having 7 to 20 carbon atoms which is substituted with an acyl group, an alicyclic hydrocarbon group having 3 to 12 carbon atoms, 10-camphoryl group or a group represented by the following Formula (II), which aliphatic hydrocarbon group, aryl group, arylalkyl group or alicyclic hydrocarbon group is unsubstituted or substituted with a group selected from a halogen atom, a halogenated alkyl group having 1 to 4 carbon atoms, an alkoxy group having 1 to 18 carbon atoms and an alkylthio group having 1 to 18 carbon atoms) 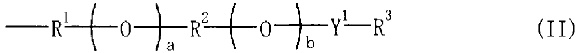 (wherein, Y¹ represents a single bond or an alkanediyl group having 1 to 4 carbon atoms; R¹ and R² each independently represent an alkanediyl group having 2 to 6 carbon atoms, a halogenated alkanediyl group having 2 to 6 carbon atoms, an arylene group having 6 to 20 carbon atoms or a halogenated arylene group having 6 to 20 carbon atoms; R³ represents a linear or branched alkyl group having 1 to 18 carbon atoms, a halogenated linear or branched alkyl group having 1 to 18 carbon atoms, an alicyclic hydrocarbon group having 3 to 12 carbon atoms, an aryl group having 6 to 20 carbon atoms, a halogenated aryl group having 6 to 20 carbon atoms, an arylalkyl group having 7 to 20 carbon atoms or a halogenated arylalkyl group having 7 to 20 carbon atoms; a and b each represent 0 or 1; and one of a and b is 1).

2. The compound according to claim 1, wherein said X is an alkyl group having 4 carbon atoms.

3. The compound according to claim 1, wherein said R is a perfluoroalkyl group having 1 to 8 carbon atoms.

4. A photoacid generator, composed of the compound according to any one of claims 1 to 3.

5. A cationic polymerization initiator, composed of the compound according to any one of claims 1 to 3.

6. A resist composition, comprising the photoacid generator according to claim 4.

7. The resist composition according to claim 6, further comprising a resist base resin, preferably wherein said resist base resin has a weight-average molecular weight (Mw) of 1,500 to 300,000.

8. The resist composition according to claim 7, wherein said photoacid generator is present in an amount of 0.01 to 20 parts by mass with respect to 100 parts by mass of the resist base resin.

9. A cationically polymerizable composition, comprising the cationic polymerization initiator according to claim 5.

10. The cationically polymerizable composition according to claim 9, further comprising a cationically polymerizable compound, preferably wherein said cationically polymerizable compound is selected from; epoxy compounds, oxetane compounds, cyclic lactone compounds, cyclic acetal compounds, cyclic thioether compounds, spiro-orthoester compounds, vinyl compounds or mixtures thereof.

11. The cationically polymerizable composition according to claim 10, wherein said cationic polymerization initiator is present in an amount of 0.01 parts by mass to 10 parts by mass with respect to 100 parts by mass of the cationically polymerizable compound.

12. A method of generating acid, comprising the steps of;
providing the compound according to any of claims 1 to 3; and
irradiating said compound with an active energy beam to generate acid.

13. A method of polymerizing a cationically polymerizable composition, comprising the steps of;
providing the compound according to any of claims 1 to 3;
providing a cationically polymerizable compound;
blending said compound according to any of claims 1 to 3 with said cationically polymerizable compound in order to form a cationically polymerizable composition; and
irradiating said cationically polymerizable composition with an active energy beam in order to induce polymerization.

14. The method according to claim 13, wherein said cationically polymerizable compound is selected from; epoxy compounds, oxetane compounds, cyclic lactone compounds, cyclic acetal compounds, cyclic thioether compounds, spiro-orthoester compounds, vinyl compounds and mixtures thereof.

## Patentansprüche

1. Eine Sulfonsäurederivatverbindung, die durch die folgende Formel (I) repräsentiert wird: 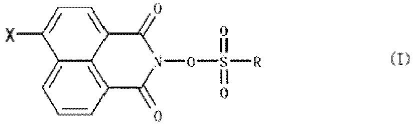 (wobei X eine geradlinige oder verzweigte Alkylgruppe mit 3 bis 8 Kohlenstoffatomen repräsentiert; und R eine aliphatische Kohlenwasserstoffgruppe mit 1 bis 18 Kohlenstoffatomen, eine Arylgruppe mit 6 bis 20 Kohlenstoffatomen, eine Arylalkylgruppe mit 7 bis 20 Kohlenstoffatomen, eine Arylgruppe mit 7 bis 20 Kohlenstoffatomen, die durch eine Acylgruppe, eine alizyklische Kohlenwasserstoffgruppe mit 3 bis 12 Kohlenstoffatomen, eine 10-Camphorylgruppe oder eine durch die folgende Formel (II) repräsentierte Gruppe substituiert wird, repräsentiert, wobei die aliphatische Kohlenwasserstoffgruppe, Arylgruppe, Arylalkylgruppe oder alizyklische Kohlenwasserstoffgruppe nicht substituiert ist oder durch eine Gruppe, die aus eine Halogenatom, einer halogenierten Alkylgruppe mit 1 bis 4 Kohlenstoffatomen, einer Alkoxygruppe mit 1 bis 18 Kohlenstoffatomen und einer Alkylthiogruppe ausgewählt wird, substituiert wird) 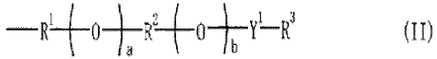 (wobei Y¹ eine Einzelbindung oder eine Alkandiylgruppe mit 1 bis 4 Kohlenstoffatomen repräsentiert; R¹ und R² jeweils unabhängig eine Alkandiylgruppe mit 2 bis 6 Kohlenstoffatomen, eine halogenierte Alkandiylgruppe mit 2 bis 6 Kohlenstoffatomen, eine Arylengruppe mit 6 bis 20 Kohlenstoffatomen oder eine halogenierte Arylengruppe mit 6 bis 20 Kohlenstoffatomen repräsentieren; R³ eine geradlinige oder verzweigte Alkylgruppe mit 1 bis 18 Kohlenstoffatomen, eine halogenierte lineare oder verzweigte Alkylgruppe mit 1 bis 18 Kohlenstoffatomen, eine alizyklische Kohlenwasserstoffgruppe mit 3 bis 12 Kohlenstoffatomen, eine Arylgruppe mit 6 bis 20 Kohlenstoffatomen, eine halogenierte Arylgruppe mit 6 bis 20 Kohlenstoffatomen, eine Arylalkylgruppe mit 7 bis 20 Kohlenstoffatomen oder eine halogenierte Arylalkylgruppe mit 7 bis 20 Kohlenstoffatomen repräsentiert; a und b jeweils 0 oder 1 repräsentieren und entweder a oder b 1 ist).

2. Die Verbindung entsprechend Anspruch 1, wobei das genannten X eine Alkylgruppe mit 4 Kohlenstoffatomen ist.

3. Die Verbindung entsprechend Anspruch 1, wobei das genannte R eine Perfluoralkylgruppe mit 1 bis 8 Kohlenstoffatomen ist.

4. Ein Fotosäuregenerator, der sich aus der Verbindung entsprechend einem der Ansprüche 1 bis 3 zusammensetzt.

5. Ein kationischer Polymerisationsaktivator, der sich aus der Verbindung entsprechend einem der Ansprüche 1 bis 3 zusammensetzt.

6. Eine Resistzusammensetzung, welche den Fotosäuregenerator entsprechend Anspruch 4 umfasst.

7. Die Resistzusammensetzung entsprechend Anspruch 6, die zudem ein Resistbasisharz umfasst, wobei das genannte Resistbasisharz bevorzugt eine gewichtete, durchschnittliche Molekülmasse (Mm) von 1500 bis 300000 hat.

8. Die Resistzusammensetzung entsprechend Anspruch 7, wobei der genannte Fotosäuregenerator in einer Menge von 0,01 bis 20 Masseteilen in Bezug auf 100 Masseteile des Resistbasisharzes vorhanden ist.

9. Eine aktionische polymerisierbare Zusammensetzung, welche den kationischen Polymerisationsaktivator entsprechend Anspruch 5 umfasst.

10. Die kationische polymerisierbare Zusammensetzung entsprechend Anspruch 9, die zudem eine kationisch polymerisierbare Verbindung umfasst, wobei die genannte kationisch polymerisierbare Verbindung bevorzugt aus Folgenden ausgewählt wird: Epoxyverbindungen, Oxetanverbindungen, zyklischen Lactonverbindungen, zyklischen Acetalverbindungen, zyklischen Thioetherverbindungen, Spiroorthoesterverbindungen, Vinylverbindungen oder Mischungen dieser.

11. Die kationische polymerisierbare Zusammensetzung entsprechend Anspruch 10, wobei der genannte kationische Polymerisationsaktivator in einer Menge von 0,01 Masseteilen bis 10 Masseteilen in Bezug auf 100 Masseteile der kationisch polymerisierbaren Verbindung vorhanden ist.

12. Ein Verfahren zur Erzeugung von Säure, das die folgenden Schritte umfasst:
Bereitstellen einer Verbindung entsprechend einem der Ansprüche 1 bis 3; und Bestrahlen der genannten Verbindung mit einem aktiven Energiestrahl zur Erzeugung von Säure.

13. Ein Verfahren zur Polymerisierung einer kationisch polymerisierbaren Zusammensetzung, das die folgenden Schritte umfasst:
Bereitstellen einer Verbindung entsprechend einem der Ansprüche 1 bis 3;
Bereitstellen einer polymerisierbaren Verbindung;
Mischen der genannten Verbindung entsprechend einem der Ansprüche 1 bis 3 mit der genannten kationisch polymerisierbaren Verbindung, um eine kationische polymerisierbare Zusammensetzung zu bilden; und
Bestrahlen der genannten kationisch polymerisierbaren Zusammensetzung mit einem aktiven Energiestrahl, um Polymerisation einzuleiten.

14. Das Verfahren entsprechend Anspruch 13, wobei die genannte kationisch polymerisierbare Verbindung aus Folgenden ausgewählt wird: Epoxyverbindungen, Oxetanverbindungen, zyklischen Lactonverbindungen, zyklischen Acetalverbindungen, zyklischen Thioetherverbindungen, Spiroorthoesterverbindungen, Vinylverbindungen oder Mischungen dieser.

## Revendications

1. Composé dérivé d'un acide sulfonique, qui est représenté par la formule (I) suivante : 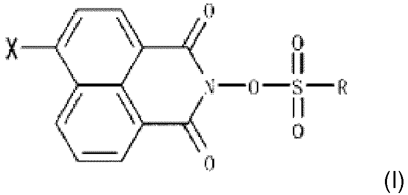 (où, X représente un groupement alkyle linéaire ou ramifié à 3 à 8 atomes de carbone ; et R représente un groupement hydrocarboné aliphatique à 1 à 18 atomes de carbone, un groupement aryle à 6 à 20 atomes de carbone, un groupement arylalkyle à 7 à 20 atomes de carbone, un groupement aryle à 7 à 20 atomes de carbone qui est substitué par un groupement acyle, un groupement hydrocarboné alicyclique à 3 à 12 atomes de carbone, un groupement 10-camphoryle ou un groupement représenté par la formule (II) suivante, ledit groupement hydrocarboné aliphatique, groupement aryle, groupement arylalkyle ou groupement hydrocarboné alicyclique étant non substitué ou substitué par un groupement sélectionné parmi un atome d'halogène, un groupement alkyle halogéné à 1 à 4 atomes de carbone, un groupement alkoxy à 1 à 18 atomes de carbone et un groupement alkylthio à 1 à 18 atomes de carbone) 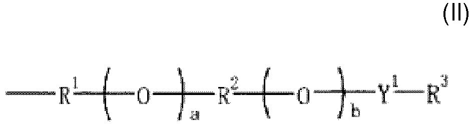 (où, Y¹ représente une simple liaison ou un groupement alkanediyle à 1 à 4 atomes de carbone ; R¹ et R² représentent chacun indépendamment un groupement alkanediyle à 2 à 6 atomes de carbone, un groupement alkanediyle halogéné à 2 à 6 atomes de carbone, un groupement arylène à 6 à 20 atomes de carbone ou un groupement arylène halogéné à 6 à 20 atomes de carbone ; R³ représente un groupement alkyle linéaire ou ramifié à 1 à 18 atomes de carbone, un groupement alkyle halogéné linéaire ou ramifié à 1 à 18 atomes de carbone, un groupement hydrocarboné alicyclique à 3 à 12 atomes de carbone, un groupement aryle à 6 à 20 atomes de carbone, un groupement aryle halogéné à 6 à 20 atomes de carbone, un groupement arylalkyle à 7 à 20 atomes de carbone ou un groupement arylalkyle halogéné à 7 à 20 atomes de carbone ; a et b prennent chacun la valeur 0 ou 1 ; et un de a et de b prend la valeur 1).

2. Composé selon la revendication 1, où ledit X est un groupement alkyle à 4 atomes de carbone.

3. Composé selon la revendication 1, où ledit R est un groupement perfluoroalkyle à 1 à 8 atomes de carbone.

4. Photogénérateur d'acide composé du composé selon l'une quelconque des revendications 1 à 3.

5. Initiateur de polymérisation cationique composé du composé selon l'une quelconque des revendications 1 à 3.

6. Composition photosensible comprenant le photogénérateur d'acide selon la revendication 4.

7. Composition photosensible selon la revendication 6 comprenant en outre une résine de base photosensible, de préférence où ladite résine de base photosensible a une masse moléculaire moyenne en poids (Mw) qui va de 1 500 à 300 000.

8. Composition photosensible selon la revendication 7, où ledit photogénérateur d'acide est présent à une quantité qui va de 0,01 à 20 parties en masse pour 100 parties en masse de la résine de base photosensible.

9. Composition cationiquement polymérisable qui comprend l'initiateur de polymérisation cationique selon la revendication 5.

10. Composition cationiquement polymérisable selon la revendication 9 comprenant en outre un composé cationiquement polymérisable, de préférence où ledit composé cationiquement polymérisable est sélectionné parmi les suivants : composés époxydes, composés oxétanes, composés lactones cycliques, composés acétals cycliques, composés thioéthers cycliques, composés spiro-orthoesters, composés vinyliques ou mélanges de ceux-ci.

11. Composition cationiquement polymérisable selon la revendication 10, où ledit initiateur de polymérisation cationique est présent à une quantité qui va de 0,01 partie en masse à 10 parties en masse pour 100 parties en masse de composé cationiquement polymérisable.

12. Procédé de production d'un acide comprenant les étapes consistant à :
se munir du composé selon l'une quelconque des revendications 1 à 3 ; et
irradier ledit composé avec un faisceau d'énergie active pour produire un acide.

13. Procédé de polymérisation d'une composition cationiquement polymérisable, comprenant les étapes consistant à :
se munir du composé selon l'une quelconque des revendications 1 à 3 ;
se munir d'un composé cationiquement polymérisable ;
mélanger ledit composé selon l'une quelconque des revendications 1 à 3 et le composé cationiquement polymérisable pour former une composition cationiquement polymérisable ; et
irradier la composition cationiquement polymérisable avec un faisceau d'énergie active pour induire la polymérisation.

14. Procédé selon la revendication 13, où le composé cationiquement polymérisable est sélectionné parmi les suivants : composés époxydes, composés oxétanes, composés lactones cycliques, composés acétals cycliques, composés thioéthers cycliques, composés spiro-orthoesters, composés vinyliques ou mélanges de ceux-ci.
